Europäisches Patentamt

European Patent Office

Office européen des brevets

(11) **EP 0 706 989 B1**

(19)

(12) **EUROPÄISCHE PATENTSCHRIFT**

(45) Veröffentlichungstag und Bekanntmachung des Hinweises auf die Patenterteilung:
**06.05.1999 Patentblatt 1999/18**

(51) Int. Cl.$^6$: **C07C 67/58**, C07C 69/54

(21) Anmeldenummer: **95115502.7**

(22) Anmeldetag: **30.09.1995**

(54) **Verfahren zur Abtrennung eines (Meth)acrylsäurediesters eines C4- bis C6-Alkandiols aus einem Gemisch, das im wesentlichen aus dem C4- bis C6-Alkandiol, dem (Meth)acrylsäuremonoester des C4- bis C6-Alkandiols und dem (Meth)acrylsäurediester des C4- bis C6-Alkandiols besteht**

Process for the separation of diester of (meth)acrylic acid with C4 to C6 alcanediol from a mixture containing mainly the C4 to C6 alcanediol and the diester of (meth)acrylic acid with C4 to C6 alcanediol

Procédé de séparation d'un diester d'acide (méth)acrylique d'un alcanediol en C4 à C6 d'un mélange constitué essentiellement de l'alcanediol en C4 à C6 et du diester d'acide (méth)acrylique et de l'alcanediol en C4 à C6

(84) Benannte Vertragsstaaten:
**BE DE DK ES FR GB IT NL**

(30) Priorität: **11.10.1994 DE 4436242**

(43) Veröffentlichungstag der Anmeldung:
**17.04.1996 Patentblatt 1996/16**

(73) Patentinhaber:
**BASF AKTIENGESELLSCHAFT**
**67056 Ludwigshafen (DE)**

(72) Erfinder:
• **Dockner, Toni, Dr.**
  **D-67149 Meckenheim (DE)**
• **Lermer, Helmut, Dr.**
  **D-67067 Ludwigshafen (DE)**
• **Rauh, Ulrich**
  **D-67117 Limburgerhof (DE)**
• **Nestler, Gerhard, Dr.**
  **D-67061 Ludwigshafen (DE)**

(56) Entgegenhaltungen:
**EP-A- 0 465 853**          **DE-A- 1 518 572**

Bemerkungen:
Die Akte enthält technische Angaben, die nach dem Eingang der Anmeldung eingereicht wurden und die nicht in dieser Patentschrift enthalten sind.

**Beschreibung**

Die vorliegende Erfindung betrifft ein Verfahren zum Abtrennen eines (Meth)acrylsäurediesters eines $C_4$- bis $C_6$-Alkandiols aus einem Gemisch, das im wesentlichen aus dem $C_4$- bis $C_6$-Alkandiol, dem (Meth)acrylsäuremonoester des $C_4$- bis $C_6$-Alkandiols und dem (Meth)acrylsäurediester des $C_4$- bis $C_6$-Alkandiol besteht, durch Extraktion mit einem organischen Lösungsmittel im Beisein von Wasser.

"(Meth)acrylsäure" wird als verkürzte Schreibweise verwendet und steht für Acrylsäure oder Methacrylsäure.

(Meth)acrylsäurediester eines $C_4$- bis $C_6$-Alkandiols bilden unvermeidbare Nebenprodukte bei der Herstellung von (Meth)acrylsäuremonoestern von $C_4$- bis $C_6$-Alkandiolen durch direkte Veresterung von (Meth)acrylsäure mit den entsprechenden Alkandiolen. Sowohl die (Meth)acrylsäuremonoester von $C_4$- bis $C_6$-Alkandiolen als auch die (Meth)acrylsäurediester von $C_4$- bis $C_6$-Alkandiolen stellen aufgrund ihrer Bifunktionalität interessante Ausgangsverbindungen dar.

Beispielsweise eignen sich die (Meth)acrylsäuremonoester von $C_4$- bis $C_6$-Alkandiolen als vinylisch ungesättigte Comonomere in z.B. als Bindemitteln geeigneten, durch radikalische Polymerisation erzeugten Polymerisaten. Als alkoholische Verbindungen eignen sie sich aber auch z.B. für polymeranaloge Umsetzungen (z.B. Kondensations- oder Additionsreaktionen). Desgleichen eignen sich (Meth)acrylsäurediester von $C_4$- bis $C_6$-Alkandiolen als zweifach vinylisch ungesättigte Verbindungen z.B. als Comonomere von in wäßrigem Medium dispergierten Polymerisaten, um letztere zu vernetzen. Bezüglich der vorgenannten Verwendungszwecke ist es erforderlich, daß sowohl die (Meth)acrylsäuremonoester von $C_4$- bis $C_6$-Alkandiolen als auch die (Meth)acrylsäurediester von $C_4$- bis $C_6$-Alkandiolen in hoher Reinheit verfügbar sind. In Ullmanns Encyclopädie der technischen Chemie, Polyacryl-Verbindungen, Bd. 19, 4. Auflage, Verlag Chemie (1980), S. 10, findet man sowohl (Meth)acrylsäuremonoester von $C_4$- bis $C_6$-Alkandiolen als auch (Meth)acrylsäurediester von $C_4$- bis $C_6$-Alkandiolen charakterisiert.

Die Herstellung von (Meth)acrylsäuremonoestern von $C_4$- bis $C_6$-Alkandiolen durch direkte Veresterung von (Meth)acrylsäure mit den entsprechenden Alkandiolen in Gegenwart saurer Veresterungskatalysatoren und unter Entfernung des entstehenden Wassers durch azeotrope Destillation mit einem geeigneten Schleppmittel ist z.B. aus der DE-PS 15 18 572 und der EP-A 465 853 bekannt.

Da Alkohole unter dem Einfluß der sauren Veresterungskatalysatoren in der Regel zu Nebenreaktionen wie Etherbildung neigen, erfolgt die Veresterung vorzugsweise nur mit einem geringen Überschuß des Alkandiols. Unter solchen Bedingungen erfolgt normalerweise in nicht unerheblichen Mengen die Bildung der entsprechenden Diester, so daß als Produktgemisch in der Regel ein solches erhalten wird, das im wesentlichen aus

- Schleppmittel,
- Alkandiol,
- Monoester und
- Diester

besteht und aus dem der gewünschte Monoester abgetrennt werden muß.

Nachteilig für die geforderte Trennaufgabe ist, daß

- die Siedepunkte von Alkandiol, Monoester und Diester selbst unter reduziertem Druck bei erhöhten Temperaturen liegen;

- die Siedepunkte von Alkandiol, Monoester und Diester sehr nahe beieinander liegen;

- die vinylisch ungesättigten Monoester und Diester insbesondere in kondensierter Phase und bei erhöhter Temperatur eine erhöhte Polymerisationsneigung aufweisen.

Im Fall von Acrylsäure als Ausgangssäure und 1,4-Butandiol als Alkandiol betragen die relevanten Siedepunkte bei Normaldruck (1 atm) beispielsweise:

- 1,4-Butandiol: 230°C (Römpp Chemie Lexikon, 9. Auflage, Thieme Verlag, Stuttgart, 1989);

- 1,4-Butandiolmonoacrylat: ca. 230°C (Technische Information TI/ED 1331 d aus 1987, der BASF Aktiengesellschaft);

- 1,4-Butandioldiacrylat: ca. 225°C (Ullmanns Encyklopädie der technischen Chemie, Bd. 19, Verlag Chemie, Weinheim, 1980, S. 9).

Unter diesen Voraussetzungen scheidet eine rektifikative Auftrennung unter ökonomischen Gesichtspunkten faktisch aus (vgl. auch DE-OS 42 28 397).

In der DE-PS 15 18 572 wird zur Auftrennung des Produktgemisches empfohlen, selbiges mit Wasser zu versetzen und im Beisein der wäßrigen Phase zunächst den Diester mit einem Extraktionsmittel zu extrahieren. Normalerweise wird als Extraktionsmittel ein solches verwendet, das gleichzeitig als Schleppmittel für die Veresterung geeignet ist, um die den Diester enthaltende organische Extraktionsphase unmittelbar in die Veresterung rückführen zu können. Anderenfalls wäre das Schleppmittel vor der extraktiven Abtrennung des Diesters z.B. destillativ abzutrennen. Selbstverständlich kann der Diester bei Bedarf auch aus der organischen Extraktionsphase isoliert werden (z.B. destillative Abtrennung des Extraktionsmittels). Das Beisein der wäßrigen Phase ist erforderlich, um zu vermeiden, daß die organische Extraktionsphase auch nennenswerte Mengen an Monoester aufnimmt. Letzterer fällt so als Bestandteil der wäßrigen Phase an und kann aus dieser in einem darauffolgenden weiteren Extraktionsschritt gewonnen werden.

Nachteilig an der Lehre gemäß der DE-PS 15 18 572 ist, daß sie empfiehlt, das Alkandiol, Monoester und Diester enthaltende Gemisch zunächst mit Wasser zu verdünnen und das resultierende wäßrige Gemisch anschließend in einer Extraktionskolonne im Gegenstrom zum Extraktionsmittel zu führen. Die im Rahmen dieser Verfahrensweise erforderliche Menge Wasser wird als das fünffache (5-fache) der Menge des aufzutrennenden Gemisches angegeben.

In der EP-A 465 853 wird empfohlen, die Veresterung im Beisein von erheblichen Mengen an zugesetztem vorgebildetem Diester durchzuführen, um auf diese Weise den Anteil an nicht umgesetztem Alkandiol im Produktgemisch zu minimieren. Ferner empfiehlt die EP-A 465 853 aus dem Produktgemisch die katalytisch wirksame Säure auszuwaschen, danach in Abwesenheit von Wasser die Diesterverbindung mit entsprechenden Extraktionsmitteln, wie sie auch die DE-PS 15 18 572 empfiehlt, direkt zu extrahieren. Nachteilig an dieser Verfahrensweise ist jedoch, daß auf diese Weise nicht unerhebliche Mengen an Monoester in das Extraktionsmittel übergehen, die im nachhinein in einem zweiten Schritt aus selbigem mit Wasser ausgewaschen werden müssen.

Die Aufgabe der vorliegenden Erfindung bestand daher darin, ein Verfahren zum Abtrennen eines (Meth)acrylsäurediesters eines $C_4$- bis $C_6$-Alkandiols aus einem Gemisch, das im wesentlichen aus dem $C_4$- bis $C_6$-Alkandiol, dem (Meth)acrylsäuremonoester des $C_4$- bis $C_6$-Alkandiols und dem (Meth)acrylsäurediester des $C_4$- bis $C_6$-Alkandiols besteht, durch Extraktion mit einem organischen Lösungsmittel im Beisein von Wasser zur Verfügung zu stellen, das bei Anwendung einer reduzierten Wassermenge bereits eine voll befriedigende Trennschärfe zwischen (Meth)acrylsäurediester und (Meth)acrylsäuremonoester sowie Alkandiol gewährleistet.

Demgemäß wurde ein Verfahren zum Abtrennen eines (Meth)acrylsäurediesters eines $C_4$- bis $C_6$-Alkandiols aus einem Gemisch (Extraktionsgut), das im wesentlichen aus dem $C_4$- bis $C_6$-Alkandiol, dem (Meth)acrylsäuremonoester des $C_4$- bis $C_6$-Alkandiols und dem (Meth)acrylsäurediester des $C_4$- bis $C_6$-Alkandiols besteht durch Extraktion mit einem organischen Lösungsmittel (Extraktionsmittel) im Beisein von Wasser gefunden, das dadurch gekennzeichnet ist, daß man im Mittelteil einer Extraktionskolonne das den Diester enthaltende Gemisch kontinuierlich aufgibt und im übrigen in der Extraktionskolonne Wasser und das organische Extraktionsmittel mit der Maßgabe kontinuierlich im Gegenstrom führt, daß das Extraktionsmittel, Extraktionsgut und Wasser der Extraktionskolonne in solchen Gewichtsmengen zugeführt werden, daß gilt:

$$\text{Gewicht zugeführtes Wasser/Gewicht zugeführtes Extraktionsgut} = 2 \text{ bis } 4$$

und

$$\text{Gewicht zugeführtes Extraktionsmittel/Gewicht zugeführtes Extraktionsgut} = 1 \text{ bis } 4.$$

Normalerweise ist Wasser spezifisch schwerer als das Extraktionsmittel und wird daher am Kopf der Kolonne aufgegeben, während das spezifisch leichtere Extraktionsmittel ins untere Ende der Extraktionskolonne gepumpt wird. Unter den vorgenannten Bedingungen kann am Kolonnenkopf die den (Meth)acrylsäurediester des $C_4$- bis $C_6$-Alkandiols enthaltende organische Phase (das Extrakt) kontinuierlich entnommen werden, während dem Kolonnensumpf kontinuierlich die das Alkandiol und den Monoester enthaltende wäßrige Phase (das Raffinat) entnommen werden kann.

Als Extraktionskolonnen kommen im Prinzip all jene in Betracht, die sich auch für Gegenstromdestillationsverfahren eignen. D.h. Boden-, Füllkörper- und Rotationskolonnen (Rühr-, Drehscheiben und Quirlkolonnen). Vorzugsweise wird man Füllkörperkolonnen anwenden unter denen wiederum jene bevorzugt sind, die als Füllkörper Pallringe oder Raschigringe enthalten. Die Füllkörper befinden sich in der Regel auf einem Tragerost und die Flüssigphasen werden in der Regel mittels Düsenrohren über den Querschnitt der Kolonne verteilt aufgebracht. Die spezifisch leichtere Phase steigt in der spezifisch schwereren Phase auf. Die Extraktionskolonne kann sowohl gepulst, als auch ungepulst betrieben werden.

Unter dem Mittelteil der Extraktionskolonne wird hier der Teil verstanden, der von der Mitte des extraktiv wirksamen Teils (der extraktiv wirksamen Gesamtstrecke) der Extraktionskolonne aus betrachtet noch 50 % des extraktiv wirksamen oberen Teils und 50 % des extraktiv wirksamen unteren Teils der Extraktionskolonne umfaßt.

Als Extraktionsmittel werden mit Vorteil aliphatische oder cycloaliphatische Kohlenwasserstoffe mit bei Normaldruck (1 atm) im Bereich von 65 bis 120°C liegenden Siedepunkten angewendet. Beispiele hierfür sind Pentane, Cyclopentan, Hexane, Cyclohexan, Heptane, Cycloheptan, Methylcyclohexan und Octane. In der Regel verfügt die Extraktionskolonne über 2 bis 10, vorzugsweise 4 bis 6, theoretische Trennstufen.

Üblicherweise wird das erfindungsgemäße Verfahren bei Normaldruck (1 atm) und einer bei 10 bis 60°C, vorzugsweise 20 bis 40°C, liegenden Extraktionstemperatur durchgeführt.

Mit Vorteil wird das erfindungsgemäße Verfahren angewendet, wenn es sich bei dem abzutrennenden (Meth)acrylsäurediester um den Acrylsäurediester von 1,4-Butandiol oder 1,6-Hexandiol handelt, der vom entsprechenden Acrylsäuremonoester im Gemisch mit dem entsprechenden Alkandiol abzutrennen ist.

Besonders günstig ist die Anwendung des erfindungsgemäßen Verfahrens dann, wenn das Extraktionsgut als Produktgemisch einer klassischen Veresterung von (Meth)acrylsäure mit einem $C_4$- bis $C_6$-Alkandiol in Gegenwart saurer Veresterungskatalysatoren und unter Entfernung des entstehenden Wassers durch azeotrope Destillation mit einem der vorgenannten günstigen Extraktionsmittel als Schleppmittel anfällt.

Zweckmäßigerweise wird zur Veresterung ein Ausgangsmolverhältnis von (Meth)acrylsäure zu Alkandiol von 1:1 bis 1:3 gewählt. Als Alkandiol kommen insbesondere 1,6-Hexandiol und 1,4-Butandiol in Betracht. Als Veresterungskatalysatoren wendet man in der Regel Säuren wie p-Toluolsulfonsäure, Schwefelsäure, Methansulfonsäure und Phosphorsäure an. Bezogen auf die zu veresternde Säure werden normalerweise 0,1 bis 3 Gew.-% an saurem Katalysator angewendet. Die Veresterungstemperatur liegt mit Vorteil bei einer Temperatur von 80 bis 140°C. Sie kann unter Unterdruck oder Überdruck durchgeführt werden. Üblich ist jedoch Normaldruck (1 atm). Vorteilhaft führt man die Veresterung kontinuierlich in einer Kaskade aus 2 bis 4 Reaktionsgefäßen durch, von denen das erste Gefäß mit Zulaufleitungen verbunden ist, während aus den folgenden Gefäßen lediglich das Reaktionswasser und schließlich das Produktgemisch entfernt wird. Bevorzugtes Schleppmittel für das zu entfernende Wasser ist Cyclohexan. Es ist gleichzeitig das vorzugsweise zur Extraktion des Diesters eingesetzte Mittel. Die Menge des Schleppmittels wird zweckmäßig so bemessen, daß der Reaktoraustrag aus dem letzten Gefäß der Kaskade 20 bis 40 Gew.-% Schleppmittel umfaßt. Das im Rahmen der erfindungsgemäßen Extraktion abgetrennte, den Diester enthaltende Extrakt wird mit Vorteil in die Veresterungskaskade zurückgeführt und dabei auf die verschiedenen Reaktionsgefäße entsprechend der aus dem jeweiligen Gefäß zu entfernenden Wassermenge verteilt.

D.h. mit Vorteil eignet sich das erfindungsgemäße Verfahren, wenn das Extraktionsgut besteht aus

- 5 bis 20 Gew.-% (Meth)acrylsäurediester des $C_4$- bis $C_6$-Alkandiols,

- 20 bis 40 Gew.-% (Meth)acrylsäuremonoester des $C_4$- bis $C_6$-Alkandiols,

- 15 bis 25 Gew.-% $C_4$- bis $C_6$-Alkandiol,

- 20 bis 50 Gew.-% Extraktionsmittel und

- $\leq$ 5 Gew.-% sonstige Verbindungen (z.B. (Meth)acrylsäure, katalytische Säure, Wasser, Polymerisationsinhibitor, Nebenprodukte).

Dies gilt insbesondere dann, wenn es sich um das Produktgemisch der Veresterung von Acrylsäure mit 1,4-Butandiol oder 1,6-Hexandiol handelt.

Extraktionsmittel, Extraktionsgut und Wasser werden im Rahmen des erfindungsgemäßen Verfahrens der Extraktionskolonne in solchen Gewichtsmengen zugeführt, daß gilt:

Gewicht zugeführtes Wasser / Gewicht zugeführtes Extraktionsgut = 2 bis 4, vorzugsweise 2 bis 3;

Gewicht zugeführtes Extraktionsmittel / Gewicht zugeführtes Extraktionsgut = 1 bis 4, vorzugsweise 2 bis 3.

Bemerkenswerterweise erreicht das erfindungsgemäße Verfahren eine voll befriedigende Trennschärfe zwischen Diester und Monoester. Ferner überrascht, daß im Rahmen des erfindungsgemäßen Verfahrens keinerlei Phasentrennprobleme auftreten.

Vorzugsweise handelt es sich bei der erfindungsgemäß zu verwendenden Extraktionskolonne um eine Pulsationskolonne, d.h. die spezifisch leichtere Phase wird am unteren Ende der Extraktionskolonne in pulsierender Weise mittels einer Pulsationspumpe in die Exraktionskolonne gedrückt.

In der Regel fällt bei dem erfindungsgemäßen Verfahren ein Extrakt an, das weniger als 0,1 Gew.-% (Meth)acrylsäuremonoester des $C_4$- bis $C_6$-Alkandiols enthält. D.h. der (Meth)acrylsäuremonoester des $C_4$- bis $C_6$-

Alkandiols befindet sich ebenso wie das Alkandiol selbst praktisch ausschließlich in der wäßrigen Phase.

Aus selbiger läßt sich der (Meth)acrylsäuremonoester des $C_4$- bis $C_6$-Alkandiols z.B. durch eine nachfolgende Extraktion mittels Methylenchlorid, einem Ester aus $C_1$- bis $C_4$-Alkancarbonsäuren und $C_1$- bis $C_5$-Alkandiolen oder einem einfachen bzw. gemischten, 4 bis 8 C-Atome aufweisenden Dialkylether extraktiv abtrennen. Durch Anwendung nachfolgender destillativer Operationen wird der reine (Meth)acrylsäuremonoester des $C_4$- bis $C_6$-Alkandiols gewonnen. Bemerkenswerterweise gelingt die erfindungsgemäße Extraktion des (Meth)acrylsäurediesters selektiver, wenn das Produktgemisch der (Meth)acrylsäuremonoesterherstellung erst nach Abtrennung des Diesters neutralisiert wird. Selbstverständlich wird der erfindungsgemäße Verfahrens schritt ebenso wie alle übrigen Verfahrensschritte auf dem Weg zum (Meth)acrylsäuremonoester eines $C_4$- bis $C_6$-Alkandiols im Beisein üblicher Mengen üblicher Polymerisationsinhibitoren ausgeführt. Dies war auch im nachfolgenden Beispiel so. Als solchermaßen zu verwendende Polymerisationsinhibitoren kommen insbesondere in Betracht Phenothiazin, Hydrochinon sowie Hydrochinonmonomethylether.

Beispiel

Einer mit Raschigringen gefüllten Pulsationsfüllkörperkolonne, die vier (4) theoretische Trennstufen aufwies, wurden im Mittelteil pro Stunde 1 400 kg des nachfolgenden Gemisches zugeführt, das im Rahmen einer klassischen Veresterung von Acrylsäure mit 1,4-Butandiol zur Herstellung des Monoesters 1,4-Butandiolmonoacrylat angefallen war:

13 Gew.-%   1,4-Butandioldiacrylat,
29 Gew.-%   1,4-Butandiolmonoacrylat,
19 Gew.-%   1,4-Butandiol,
35 Gew.-%   Cyclohexan und
5 Gew.-%     sonstige Verbindungen.

Am Kopf der Kolonne wurden pro Stunde 3 600 kg Wasser aufgegeben. Am Fuß der Kolonne wurden über eine Pulsationspumpe je Stunde 3 300 kg Cyclohexan zugeführt.

Die Extraktionstemperatur betrug 25°C.

Als Extrakt wurden pro Stunde 4 000 kg einer Cyclohexanphase entnommen, die enthielt:

4,5 Gew.-%      1,4-Butandioldiacrylat und
0,05 Gew.-%   1,4-Butandiolmonoacrylat.

Als Raffinat wurden pro Stunde 4 300 kg einer wäßrigen Phase entnommen, die enthielt:

6,2 Gew.-%     1,4-Butandiol,
8,9 Gew.-%     1,4-Butandiolmonoacrylat,
0,04 Gew.-%   1,4-Butandioldiacrylat.

**Patentansprüche**

1.   Verfahren zum Abtrennen eines (Meth)acrylsäurediesters eines $C_4$- bis $C_6$-Alkandiols aus einem Gemisch (Extraktionsgut), das im wesentlichen aus dem $C_4$- bis $C_6$-Alkandiol, dem (Meth)acrylsäuremonoester des $C_4$- bis $C_6$-Alkandiols und dem (Meth)acrylsäurediester des $C_4$- bis $C_6$-Alkandiols besteht, durch Extraktion mit einem organischen Losungsmittel (Extraktionsmittel) im Beisein von Wasser, dadurch gekennzeichnet, daß man im Mittelteil einer Extraktionskolonne das den Diester enthaltende Gemisch kontinuierlich aufgibt und im übrigen in der Extraktionskolonne Wasser und das organische Lösungsmittel mit der Maßgabe kontinuierlich im Gegenstrom führt, daß das Extraktionsmittel, Extraktionsgut und Wasser der Extraktionskolonne in solchen Gewichtsmengen zugeführt werden, daß gilt:

Gewicht zugeführtes Wasser / Gewicht zugeführtes Extraktionsgut = 2 bis 4

und

Gewicht zugeführtes Extraktionsmittel / Gewicht zugeführtes Extraktionsgut = 1 bis 4.

2.   Verfahren nach Anspruch 1, dadurch gekennzeichnet, daß als Extraktionsmittel aliphatischer oder cycloaliphatischer Kohlenwasserstoff eingesetzt wird, dessen Siedepunkt bei einem Druck von 1 atm 65 bis 120°C beträgt.

3.  Verfahren nach Anspruch 1 oder 2, dadurch gekennzeichnet, daß als Extraktionsmittel Cyclohexan verwendet wird.

4.  Verfahren nach Anspruch 1 bis 3, dadurch gekennzeichnet, daß das Extraktionsgut besteht aus

    -   5 bis 20 Gew.-% (Meth)acrylsäurediester des $C_4$- bis $C_6$-Alkandiols,
    -   20 bis 40 Gew.-% (Meth)acrylsäuremonoester des $C_4$- bis $C_6$-Alkandiols,
    -   15 bis 25 Gew.-% $C_4$- bis $C_6$-Alkandiol,
    -   20 bis 50 Gew.-% Extraktionsmittel und
    -   $\leq$ 5 Gew.-% sonstige Verbindungen.

5.  Verfahren nach Anspruch 1 bis 4, dadurch gekennzeichnet, daß es sich bei dem abzutrennenden (Meth)acrylsäurediester um den Acrylsäurediester von 1,4-Butandiol oder 1,6-Hexandiol handelt, der vom entsprechenden Acrylsäuremonoester im Gemisch mit dem entsprechenden Alkandiol abzutrennen ist.

6.  Verfahren nach Anspruch 1 bis 5, dadurch gekennzeichnet, daß die Extraktionstemperatur 20 bis 40°C beträgt.

7.  Verfahren nach Anspruch 1 bis 5, dadurch gekennzeichnet, daß die Extraktionskolonne eine Füllkörperkolonne mit 2 bis 10, vorzugsweise 4 bis 6, theoretischen Trennstufen ist.

8.  Verfahren zur Herstellung eines (Meth)acrylsäuremonoesters eines $C_4$- bis $C_6$-Alkandiols durch Veresterung der (Meth)acrylsäure mit jenem Alkandiol in Gegenwart saurer Veresterungskatalysatoren unter Entfernung des entstehenden Wassers durch azeotrope Destillation mit einem Schleppmittel, extraktive Auftrennung des Produktgemisches mit einem Extraktionsmittel 1 in eine den als Nebenprodukt entstehenden Diester enthaltende organische Lösung sowie ein verwendetes Alkandiol und (Meth)acrylsäuremonoester enthaltende wäßrige Lösung und anschließende extraktive Abtrennung des (Meth)acrylsäuremonoester aus der verwendetes Alkandiol und (Meth)acrylsäuremonoester enthaltenden wäßrigen Lösung mit einem Extraktionsmittel 2 sowie nachfolgende Trennung von Extraktionsmittel 2 und (Meth)acrylsäuremonoester, bei dem das Extraktionsmittel 1 und das azeotrope Schleppmittel chemisch identisch sind und die den als Nebenprodukt entstehenden Diester enthaltende organische Lösung (Extrakt 1) in die Veresterung rückgeführt wird, dadurch gekennzeichnet, daß man im Mittelteil einer Extraktionskolonne das Produktgemisch kontinuierlich aufgibt und im übrigen in der Extraktionskolonne Wasser und das organische Extraktionsmittel 1 mit der Maßgabe kontinuierlich im Gegenstrom führt, daß das Produktgemisch, das organische Extraktionsmittel 1 und das Wasser der Extraktionskolonne in solchen Gewichtsmengen zugeführt werden, daß gilt:

    Gewicht zugeführtes Wasser/Gewicht zugeführtes Produktgemisch = 2 bis 4

    und

    Gewicht zugeführtes organisches Extraktionsmittel/Gewicht zugeführtes Produktgemisch = 1 bis 4.

9.  Verfahren nach Anspruch 8, dadurch gekennzeichnet, daß es sich um ein Verfahren zur Herstellung von 1,4-Butandiolmonoacrylat handelt.

## Claims

1.  A process for separating a diester of (meth)acrylic acid with a $C_4$-$C_6$-alkanediol from a mixture (extraction material) which essentially consists of the $C_4$-$C_6$-alkanediol, the monoester of (meth)acrylic acid with the $C_4$-$C_6$-alkanediol and the diester of (meth)acrylic acid with the $C_4$-$C_6$-alkanediol by extraction with an organic solvent (extracting agent) in the presence of water, wherein the diester-containing mixture is added continuously in the middle section of an extraction column and in addition water and the organic solvent are passed continuously countercurrent in the extraction column, with the proviso that the extracting agent, extraction material and water are fed to the extraction column in amounts by weight such that the following is true:

    weight of added water / weight of added extraction material = from 2 to 4

    and

    weight of added extracting agent / weight of added extraction material = from 1 to 4.

**2.** A process as claimed in claim 1, wherein the extracting agent used is an aliphatic or cycloaliphatic hydrocarbon whose boiling point is from 65 to 120°C at 1 atm.

**3.** A process as claimed in claim 1 or 2, wherein the extracting agent used is cyclohexane.

**4.** A process as claimed in any of claims 1 to 3, wherein the extraction material consists of

- from 5 to 20 % by weight of the diester of (meth)acrylic acid with a $C_4$-$C_6$-alkanediol,

- from 20 to 40 % by weight of the monoester of (meth)acrylic acid with a $C_4$-$C_6$-alkanediol,

- from 15 to 25 % by weight of the $C_4$-$C_6$-alkanediol,

- from 20 to 50 % by weight of the extracting agent and

- ≤ 5 % by weight of other compounds.

**5.** A process as claimed in any of claims 1 to 4, wherein the (meth)acrylic diester to be separated off is the diester of acrylic acid with 1,4-butanediol or 1,6-hexanediol, which is to be separated off from the corresponding acrylic monoester in the mixture with the corresponding alkanediol.

**6.** A process as claimed in any of claims 1 to 5, wherein the extraction temperature is from 20 to 40°C.

**7.** A process as claimed in any of claims 1 to 5, wherein the extraction column is a packed column having from 2 to 10, preferably from 4 to 6, theoretical plates.

**8.** A process for the preparation of a monoester of (meth)acrylic acid with a $C_4$-$C_6$-alkanediol by esterification of (meth)acrylic acid with this alkanediol in the presence of an acidic esterification catalyst with removal of the resulting water by azeotropic distillation with an entraining agent, separation of the product mixture by extraction with an extracting agent 1 in an organic solution containing the diester formed as by-product and an aqueous solution containing the alkanediol used and (meth)acrylic monoester and subsequent separation of the (meth)acrylic monoester by extraction from the aqueous solution containing the alkanediol used and (meth)acrylic monoester with an extracting agent 2 and subsequent separation of extracting agent 2 and (meth)acrylic monoester, in which the extracting agent 1 and the azeotropic entraining agent are chemically identical and the organic solution containing the diester formed as a byproduct (extract 1) is recycled to the esterification, wherein the product mixture is added continuously to the middle section of an extraction column and in addition water and the organic extracting agent 1 are passed continuously countercurrent in the extraction column, with the proviso that the product mixture, the organic extracting agent 1 and the water are fed to the extraction column in amounts by weight such that the following is true:

weight of added water/weight of added product mixture = from 2 to 4

and

weight of added organic extracting agent/weight of added product mixture = from 1 to 4.

**9.** A process as claimed in claim 8, which is a process for the preparation of 1,4-butanediol monoacrylate.

**Revendications**

**1.** Procédé de séparation d'un diester (méth)acrylique d'un alcanediol en $C_4$-$C_6$ à partir d'un mélange (matériau d'extraction), constitué essentiellement de l'alcanediol en $C_4$-$C_6$, du monoester (méth)acrylique de l'alcanediol en $C_4$-$C_6$ et du diester (méth)acrylique de l'alcanediol en $C_4$-$C_6$, par extraction à l'aide d'un solvant organique (agent d'extraction) en présence d'eau, caractérisé en ce que l'on introduit, de façon continue, dans la zone médiane d'une colonne d'extraction, le mélange contenant le diester, et on introduit dans la colonne d'extraction l'eau et le solvant organique de façon continue, à contre-courant, de sorte que l'agent d'extraction, le matériau d'extraction et l'eau soient introduits dans la colonne d'extraction dans des quantités telles que :

$$\text{poids d'eau introduit / poids de matériau d'extraction introduit} = 2 \text{ à } 4$$

et

$$\text{poids d'agent d'extraction introduit / poids de matériau d'extraction introduit} = 1 \text{ à } 4.$$

2. Procédé selon la revendication 1, caractérisé en ce que l'on utilise en tant qu'agent d'extraction un hydrocarbure aliphatique ou cycloaliphatique, dont le point d'ébullition s'élève à 65-120°C sous une pression de 1 atm.

3. Procédé selon la revendication 1 ou 2, caractérisé en ce que l'on utilise du cyclohexane en tant qu'agent d'extraction.

4. Procédé selon l'une quelconque des revendications 1 à 3, caractérisé en ce que le matériau d'extraction est constitué de

   - 5-20% en poids de diester (méth)acrylique de l'alcanediol en $C_4$-$C_6$,
   - 20-40% en poids de monoester (méth)acrylique de l'alcanediol en $C_4$-$C_6$,
   - 15 à 25% en poids de l'alcanediol en $C_4$-$C_6$,
   - 20-50% en poids d'agent d'extraction et
   - $\leq$ 5% en poids d'autres composés.

5. Procédé selon l'une quelconque des revendications 1 à 4, caractérisé en ce que le diester (méth)acrylique à séparer est le diester acrylique de 1,4-butanediol ou de 1,6-hexanediol qui doit être séparé du monoester acrylique correspondant mélangé à l'alcanediol correspondant.

6. Procédé selon l'une quelconque des revendications 1 à 5, caractérisé en ce que la température d'extraction s'élève à 20-40°C.

7. Procédé selon l'une quelconque des revendications 1 à 5, caractérisé en ce que la colonne d'extraction est une colonne à garnissage avec 2-10, de préférence 4-6 plateaux théoriques.

8. Procédé de préparation d'un monoester (méth)acrylique d'un alcanediol en $C_4$-$C_6$ par estérification de l'acide (méth)acrylique avec ledit alcanol, en présence de catalyseurs acides d'estérification avec élimination de l'eau produite par distillation azéotrope à l'aide d'un agent d'entraînement, séparation par extraction du mélange de produits à l'aide d'un agent d'extraction 1 en une solution organique, contenant en tant que sous-produit le diester correspondant, et en une solution aqueuse, contenant l'alcanediol utilisé et le monoester (méth)acrylique, suivie de la séparation par extraction du monoester (méth)acrylique à partir de la solution aqueuse contenant l'alcanediol utilisé et le monoester (méth)acrylique, à l'aide d'un agent d'extraction 2, puis séparation de l'agent d'extraction 2 et du monoester (méth)acrylique, dans lequel l'agent d'extraction 1 et l'agent d'entraînement azéotrope sont chimiquement identiques et l'on recycle dans l'étape d'estérification la solution organique (extrait 1) contenant en tant que sous-produit le diester obtenu, caractérisé en ce que l'on introduit, de façon continue, dans la zone médiane d'une colonne d'extraction, le mélange de produits, et on introduit dans la colonne d'extraction l'eau et l'agent d'extraction organique 1, de façon continue, à contre-courant, de sorte que le mélange de produits, l'agent d'extraction organique 1 et l'eau soient introduits dans la colonne d'extraction dans des quantités telles que :

$$\text{poids d'eau introduit / poids de mélange de produits introduit} = 2 \text{ à } 4$$

et

$$\text{poids d'agent d'extraction organique introduit / poids de mélange de produits introduit} = 1 \text{ à } 4.$$

9. Procédé selon la revendication 8, caractérisé en ce qu'il s'agit du procédé de préparation de monoacrylate de 1,4-butanediol.